# EUROPEAN PATENT APPLICATION

(11) **EP 4 834 409 A1**
(43) Date of publication of application: **04.11.2026**
(21) Application number: 25747631.7
(22) Date of filing: 21.01.2025
(51) Int. Cl.: A61M 31/00, A61M 11/06

(54) **PRE-FILLED NASAL SPRAY DEVICE HAVING SEALING SWITCH**

(30) Priority: 01.02.2024 CN 202410141633
(71) Applicant: Ding, Yaowu, Taixing, Jiangsu 225400 (CN)
(72) Inventor: Ding, Yaowu, Taixing, Jiangsu 225400 (CN)
(74) Representative: Lorenz Seidler Gossel Part. mbB
(86) International application number: PCT/CN2025/073582
(87) International publication number: WO 2025/162059

(57) **Abstract**

A pre-filled nasal spray device (100), comprising a syringe(110), a nasal spray nozzle (120) and a fixing base (130), wherein the fixing base (130) is mounted on the syringe (110), and the nasal spray nozzle (120) is connected to the fixing base (130), a liquid channel which extends from the internal chamber of the syringe (110) to the nasal spray nozzle (120) through the fixing base (130) being formed in the pre-filled nasal spray device (100). A sealing switch is provided in the liquid channel, and can switch between an open state and a closed state; in the open state, the internal chamber of the syringe (110) is in communication with the outside through the liquid channel, in the closed state, the internal chamber of the syringe (110) is sealed and isolated from the outside. In the pre-filled nasal spray device (100), the sealing switch has a simple structure, and can realize reliable sealing of pre-filled liquid medicine in the pre-filled nasal spray device (100).

## Description

### Technical Field

The present application relates to medical instruments, and specifically to a nasal spray device for administering medicine into a body cavity such as a nasal cavity, in particular, the nasal spray device is a pre-filled nasal spray device.

### Background Art

There are various types of spray drug delivery devices on the market, including a pre-filled nasal spray device. The pre-filled nasal spray device is one in which a liquid medicine is pre-filled into a syringe barrel of the spray drug delivery device. Thus, when administering medicine to a user's nasal cavity, there is no need to draw liquid medicine from a medicine container such as a vial, and the liquid medicine in the syringe barrel can be directly sprayed into the nasal cavity. The pre-filled nasal spray device is relatively convenient to use, and therefore its applications are becoming more and more widespread.

Since the liquid medicine to be administered to the user is pre-filled in the pre-filled nasal spray device, the sealing requirements for the pre-filled nasal spray device are particularly high, so as to ensure the quality of the liquid medicine contained in the pre-filled nasal spray device.

In other words, the sealing performance of the pre-filled nasal spray device is crucial to the quality of the liquid medicine contained therein. If the sealing performance of the pre-filled nasal spray device is poor, on the one hand, it will cause the liquid medicine filled in the device to leak or volatilize, thus reducing the amount of medicine that can be subsequently administered to the user, on the other hand, it will cause the liquid medicine to contact the external environment, leading to contamination or oxidation of the liquid medicine, thereby degrading the liquid medicine. A reduction in the amount of medicine will affect the therapeutic effect, and contamination or oxidation of the liquid medicine will not only affect the therapeutic effect but also pose potential safety hazards for the use of the medicine.

Therefore, strict control is required during the design and production process of the pre-filled nasal spray device, so that its sealing performance can reach the highest standards.

The sealing structure of prior art pre-filled nasal spray devices usually adopts a cap-type seal, that is, a protective cap is added to the pre-filled nasal spray device to isolate the interior of the pre-filled nasal spray device from the outside. Commonly used protective caps include, for example, bayonet-type caps and Luer-type caps. The disadvantage of the bayonet-type cap is that its seal is unreliable, and it is easy to slip off the pre-filled nasal spray device, thus causing sealing failure. The installation of the Luer-type cap requires relatively high professional skills and needs to be performed by personnel with rich practical experience, it must not be screwed on too loosely or too tightly. If it is screwed on too loosely, the required seal cannot be achieved; but if it is screwed on too tightly, the Luer connection structure between the cap and the pre-filled nasal spray device is also prone to slipping.

Therefore, for the pre-filled nasal spray device, there is a need for further improvement of its structure, which can achieve reliable sealing on the pre-filled nasal spray device with a simple structure.

### Summary

The present application is made to solve the problems existing in the above-mentioned prior art. The purpose of the present application is to provide a pre-filled nasal spray device with an improved structure, which includes a sealing switch with simple structure and can achieve reliable sealing for the pre-filled nasal spray device.

The present application provides a pre-filled nasal spray device, the pre-filled nasal spray device has a syringe, a nasal spray nozzle and a fixing base, the fixing base is mounted on the syringe, and the nasal spray nozzle is connected to the fixing base, wherein a liquid channel from an internal chamber of the syringe to the nasal spray nozzle through the fixing base is formed in the pre-filled nasal spray device. A sealing switch is provided in the liquid channel, the sealing switch is switchable between an open state and a closed state, in the open state, the internal chamber of the syringe communicates with the outside via the liquid channel, in the closed state, the internal chamber of the syringe is sealed and isolated from the outside.

In this pre-filled nasal spray device, the sealing switch has a simple structure, and can achieve reliable sealing for the pre-filled liquid medicine in the pre-filled nasal spray device.

In a specific structure, the sealing switch includes a sealing plug, a sealing surface is formed on the front end of the fixing base or on the hub of the syringe, the sealing plug can form a sealing fit with the sealing surface.

More specifically, the nasal spray nozzle is movable relative to the fixing base along an axial direction of the pre-filled nasal spray device between a closed position and an open position, in the closed position, the nasal spray nozzle presses the sealing plug against the sealing surface, in the open position, the nasal spray nozzle releases the pressing against the sealing plug.

Preferably, the fixing base includes a guide rail portion, a top surface of the guide rail portion includes a lowered surface and a raised surface; at least one sliding block is formed on an outer surface of an outer sleeve of the nasal spray nozzle, the sliding block rests on the top surface of the guide rail portion. The nasal spray nozzle is rotatable relative to the fixing base, so that the sliding block contacts the lowered surface and the raised surface alternatively, when the sliding block contacts the lowered surface, the nasal spray nozzle is in the closed position relative to the fixing base, when the sliding block contacts the raised surface, the nasal spray nozzle is in the open position relative to the fixing base.

In such a structure, the sealing switch can be easily opened and closed by rotating the nasal spray nozzle relative to the fixing base.

Alternatively, in another structure, the nasal spray nozzle is configured to be linearly movable relative to the fixing base along the axial direction of the pre-filled nasal spray device between the closed position and the open position.

Preferably, the sealing surface is a conical inner surface formed in the front end of the fixing base or in the hub of the syringe. The conical inner surface helps to improve the sealing performance of the fit between the sealing plug and the sealing surface.

In another specific structure, the sealing switch includes an opening, the sealing switch is movable between a closed position and an open position, in the closed position, the opening is closed, and in the open position, the opening is opened.

An exemplary embodiment of the opening is at least one side hole formed in the front end of the fixing base.

Preferably, the nasal spray nozzle includes an inner sleeve, an inner diameter of the inner sleeve of the nasal spray nozzle is set to be smaller than an outer diameter of a portion formed with the side hole of the front end of the fixing base, so that the portion formed with the side hole of the front end is in interference fit with the inner sleeve of the nasal spray nozzle, furthermore, at least one flow guide groove is formed in the inner sleeve of the nasal spray nozzle. Wherein, the nasal spray nozzle is rotatable relative to the fixing base between the closed position and the open position, in the closed position, the side hole is offset from the flow guide groove, in the open position, the side hole is aligned with the flow guide groove.

Further preferably, the nasal spray nozzle includes an inner sleeve, a large inner diameter portion and a small inner diameter portion are formed in the inner sleeve of the nasal spray nozzle, an inner diameter of the small inner diameter portion is smaller than an outer diameter of a portion formed with the side hole of the front end of the fixing base, so that the portion formed with the side hole of the front end is in interference fit with the small inner diameter portion, an inner diameter of the large inner diameter portion is larger than the outer diameter of the portion formed with the side hole of the front end of the fixing base. Wherein, the nasal spray nozzle is linearly movable relative to the fixing base along the axial direction of the pre-filled nasal spray device between the closed position and the open position, in the closed position, the portion formed with the side hole of the front end of the fixing base is in interference fit with the small inner diameter portion, in the open position, the portion of the front end of the fixing base where the side hole is formed is located within the large inner diameter portion.

Further preferably, a groove is formed in the nasal spray nozzle, and an outer peripheral flange is formed on the fixing base, when the nasal spray nozzle is connected to the fixing base, the outer peripheral flange fits into the groove, wherein the width of the groove is greater than the width of the outer peripheral flange, so as to allow a linear movement of the nasal spray nozzle relative to the fixing base.

In another specific implementation structure, the sealing switch includes a knob including a rod portion, the opening is a through hole formed in the rod portion. A transverse hole is formed in the fixing base, the transverse hole extends in a direction perpendicular to the axial direction of the pre-filled nasal spray device, the rod portion is inserted into the transverse hole of the fixing base. Wherein, the knob is rotatable between the closed position and the open position, in the closed position, the through hole is offset from a longitudinal channel of the fixing base, in the open position, the through hole is aligned with the longitudinal channel.

By rotating the knob, the sealing switch can be easily opened and closed.

Preferably, a finger rest plate is integrally formed on or connected to the nasal spray nozzle or the fixing base.

Further preferably, a stop clip is provided between the finger rest plate and a flange on a rear end of the syringe. The stop clip can prevent the syringe from moving forward relative to the fixing base or the nasal spray nozzle during storage and transportation.

Alternatively, at least one frangible member may be provided between the finger rest plate and the flange on the rear end of the syringe. In use, by pushing the syringe forward, the frangible member can rupture under the thrust force, thereby allowing the syringe to move forward.

### Brief Description of Drawings

Specific embodiments of the present application can be understood more clearly from the structures shown in the accompanying drawings, wherein:
Fig. 1 shows a cross-sectional view of the pre-filled nasal spray device according to a first embodiment of the present application, wherein the pre-filled nasal spray device is in a closed state.
Fig. 2 shows another cross-sectional view of the pre-filled nasal spray device of the first embodiment, wherein the pre-filled nasal spray device is in an open state.
Fig. 3a is a partially enlarged view of portion A1 in Fig. 1.
Fig. 3b is a partially enlarged view of portion B1 in Fig. 2.
Fig. 4a shows a perspective view of the nasal spray nozzle of the pre-filled nasal spray device of Fig. 1.
Fig. 4b shows a perspective view of the fixing base of the pre-filled nasal spray device of Fig. 1, wherein the fixing base is without a finger rest plate.
Fig. 4c shows a perspective view of the nasal spray nozzle of Fig. 4a and the fixing base of Fig. 4b assembled together.
Fig. 5 shows a cross-sectional view of a sealing plunger in the pre-filled nasal spray device of Fig. 1.
Fig. 6 shows a cross-sectional view of another structure of the fixing base, wherein the fixing base is with a finger rest plate.
Fig. 7 shows a cross-sectional view of the pre-filled nasal spray device according to a second embodiment of the present application, wherein the pre-filled nasal spray device is in a closed state.
Fig. 8 shows another cross-sectional view of the pre-filled nasal spray device of the second embodiment, wherein the pre-filled nasal spray device is in an open state.
Fig. 9a is a partially enlarged view of portion A2 in Fig. 7.
Fig. 9b is a partially enlarged view of portion B2 in Fig. 8.
Fig. 10 shows a cross-sectional view of the pre-filled nasal spray device according to a third embodiment of the present application, wherein the pre-filled nasal spray device is in a closed state.
Fig. 11 shows another cross-sectional view of the pre-filled nasal spray device of the third embodiment, wherein the pre-filled nasal spray device is in an open state.
Fig. 12a is a partially enlarged view of portion A3 in Fig. 10.
Fig. 12b is a partially enlarged view of portion B3 in Fig. 11.
Fig. 13 shows a cross-sectional view of the pre-filled nasal spray device according to a fourth embodiment of the present application, wherein the pre-filled nasal spray device is in a closed state.
Fig. 14 shows another cross-sectional view of the pre-filled nasal spray device of the fourth embodiment, wherein the pre-filled nasal spray device is in an open state.
Fig. 15a is a partially enlarged view of portion A4 in Fig. 13.
Fig. 15b is a partially enlarged view of portion B4 in Fig. 14.
Fig. 16 shows a cross-sectional view of the pre-filled nasal spray device according to a fifth embodiment of the present application, wherein the pre-filled nasal spray device is in a closed state.
Fig. 17 shows another cross-sectional view of the pre-filled nasal spray device of the fifth embodiment, wherein the pre-filled nasal spray device is in an open state.
Fig. 18a is a partially enlarged view of portion A5 in Fig. 16.
Fig. 18b is a partially enlarged view of portion B5 in Fig. 17.

### (List of Reference Numerals)

- 100: Pre-filled nasal spray device
- 110: Syringe
- 111: Hub
- 120: Nasal spray nozzle
- 121: Outer sleeve of nasal spray nozzle
- 122: Sliding block
- 123: Limiting block
- 130: Fixing base
- 131: Guide rail portion
- 132: Lowered surface
- 133: Raised surface
- 134: Conical inner surface
- 135: Liquid outlet
- 136: Finger rest plate
- 140: Sealing plug
- 141: Conical outer surface
- 200: Pre-filled nasal spray device
- 210: Syringe
- 220: Nasal spray nozzle
- 221: Inner sleeve of nasal spray nozzle
- 222: Flow guide groove
- 230: Fixing base
- 231: Side hole
- 300: Pre-filled nasal spray device
- 310: Syringe
- 320: Nasal spray nozzle
- 321: Inner sleeve of nasal spray nozzle
- 322: Large inner diameter portion
- 323: Small inner diameter portion
- 324: Groove
- 325: Finger rest plate
- 330: Fixing base
- 331: Side hole
- 332: Outer peripheral flange
- 350: Stop clip
- 400: Pre-filled nasal spray device
- 410: Syringe
- 420: Nasal spray nozzle
- 421: Inner sleeve of nasal spray nozzle
- 422: Groove
- 430: Fixing base
- 431: Protruding ring
- 440: Sealing plug
- 500: Pre-filled nasal spray device
- 510: Syringe
- 520: Nasal spray nozzle
- 521: Side opening
- 530: Fixing base
- 531: Transverse hole
- 532: Longitudinal channel
- 540: Knob
- 541: Rod portion
- 542: Through hole
- 543: Protruding ring

### Detailed Description

To facilitate understanding of the present application, specific embodiments of the pre-filled nasal spray device of the present application will be described in detail below with reference to the accompanying drawings. It should be understood that what is shown in the drawings are merely preferred embodiments of the present application, and they should not be construed as limiting the scope of the present application. Based on the embodiments shown in the drawings, those skilled in the art can make various obvious modifications, variations, and equivalent substitutions to the present application, and under the premise of no conflict, the technical features of the different embodiments described below can be arbitrarily combined with each other, and all these fall within the scope of the present application.

In the following description of the pre-filled nasal spray device, such terms as "front", "rear", "upper", and "lower" used to indicate directions are based on the orientation of the pre-filled nasal spray device as shown in the drawings, the orientation of the pre-filled nasal spray device shown in the drawings takes into account the orientation of the pre-filled nasal spray device when in use, wherein "front" and "upper" refer to the upper side or upper end in the drawing, which corresponds to the side or end of the pre-filled nasal spray device close to the person to be administered with medicine during use, "rear" and "lower" refer to the lower side or lower end in the drawing, which corresponds to the side or end of the pre-filled nasal spray device away from the person to be administered with medicine during use.

### <First Embodiment>

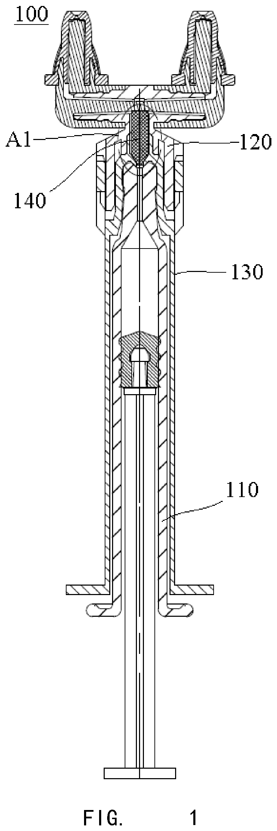

Figs. 1 - 6 show the structure of the pre-filled nasal spray device 100 and its components according to the first embodiment of the present application.

The pre-filled nasal spray device 100 includes a syringe 110, a nasal spray nozzle 120, and a fixing base 130. The nasal spray nozzle 120 is relatively movably connected to the fixing base 130, the specific connection structure will be described in more detail below. The fixing base 130 is fixed to a hub 111 of the pre-filled nasal spray device 100 at the front end of the syringe 110 by a structure such as a snap fit. Here, the syringe 110 may be a glass syringe or a plastic syringe, all of which are within the scope of the present application.

As shown in the figures, a fluid channel, from the internal chamber of the syringe 110 through the fixing base 130 to the internal chamber of the nasal spray nozzle 120, is formed in the pre-filled nasal spray device 100, the liquid medicine pre-filled in the syringe 110 can flow through this fluid channel to the nozzle of the nasal spray nozzle 120 and then be sprayed out.

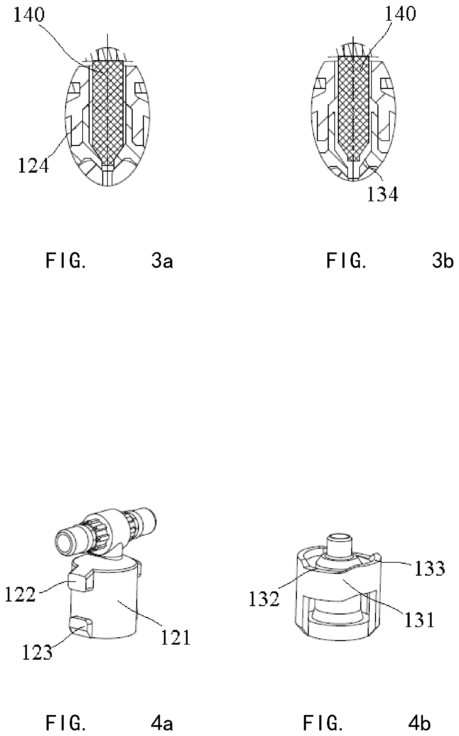

Fig. 4a shows a schematic perspective view of the nasal spray nozzle 120 of the pre-filled nasal spray device 100. The exemplary nasal spray nozzle 120 shown in the figure has a three-way structure, on which two nozzles can be connected. However, those skilled in the art will appreciate that the nasal spray nozzle 120 can also be configured as a single-head structure, which includes only one nozzle, and this also falls within the scope of the present application.

The nasal spray nozzle 120 has an outer sleeve 121, at least one sliding block 122 is formed on an outer surface of the outer sleeve 121. Preferably, two sliding blocks 122 are formed on the outer surface of the outer sleeve 121, and they are arranged 180° apart from each other. Preferably, at least one limiting block 123 is also formed on the outer surface of the outer sleeve 121, the limiting block 123 cooperates with a guide rail portion 131 of the fixing base 130, which will be described below, to achieve a relatively movable connection between the nasal spray nozzle 120 and the fixing base 130.

Fig. 4b shows a schematic perspective view of the fixing base 130 of the pre-filled nasal spray device 100. The fixing base 130 includes a guide rail portion 131 disposed on its outer periphery, the guide rail portion 131 has a guide surface at its top, the guide surface includes a lowered surface 132 and a raised surface 133.

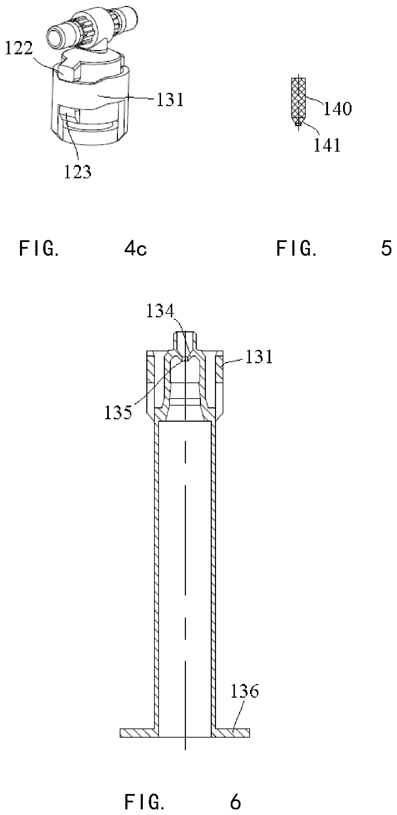

Fig. 4c shows a perspective view of the nasal spray nozzle 120 and the fixing base 130 assembled together. When assembled together, the sliding blocks 122 on the nasal spray nozzle 120 rest on the guide surface of the guide rail portion 131. The limiting blocks 123 are located below the guide rail portion 131, and cooperate with the lower surface of the guide rail portion 131 to prevent the nasal spray nozzle 120 from being separated from the fixing base 130 upwards.

With further reference to Fig. 6, it can be seen that a conical inner surface 134 is formed at the front end of the fixing base 130, the conical inner surface 134 is close to the liquid outlet 135 of the fixing base 130, in an assembled state, the liquid outlet 135 can communicate with a hole on the hub 111 of the syringe 110. The lower end of the sealing plug 140 (see Fig. 5) can form a sealing fit with the conical inner surface 134, thereby blocking the liquid outlet 135, and further preventing the liquid medicine pre-filled in the syringe 110 from leaking or contacting the external environment. It can be seen that the sealing plug 140 cooperates with the conical inner surface 134 in the fixing base 130 to form a sealing switch, and the sealing switch is disposed in the above-mentioned fluid channel.

Preferably, in the structure shown in Fig. 5, a conical outer surface 141 is formed at the lower end of the sealing plug 140, the conical outer surface 141 is shaped to match the conical inner surface 134 in the fixing base 130. Thus, when the sealing plug 140 is in sealing fit on the conical inner surface 134, a face seal is formed between the sealing plug 140 and the fixing base 130, thereby enabling a more reliable seal.

Additionally, preferably, as shown in Fig. 6, a finger rest plate 136 is also provided on the fixing base 130, the finger rest plate 136 is, for example, integrally formed at the lower end (or rear end) of the fixing base 130. Of course, the finger rest plate 136 may also be formed at other suitable positions on the fixing base 130. Furthermore, the finger rest plate 136 may also be formed separately and then connected to an appropriate position on the fixing base 130.

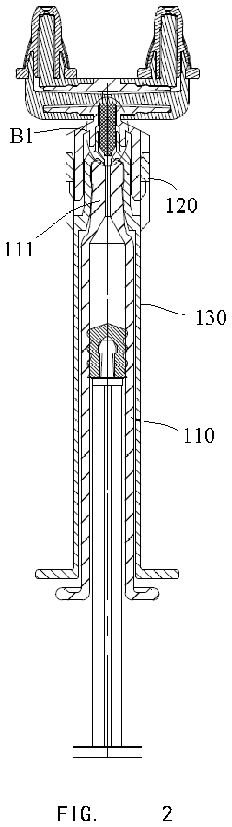

Returning to Fig. 1 and Fig. 2, and with reference to Fig. 3a and Fig. 3b, the operating principle of the pre-filled nasal spray device 100 of the first embodiment will be described.

The pre-filled nasal spray device 100 shown in Fig. 1 is in a closed state, in this state, operations such as storage and transportation of the pre-filled nasal spray device 100 can be performed. At this time, the sliding block 122 of the nasal spray nozzle 120 mates with the lowered surface 132 of the guide surface of the guide rail portion 131 of the fixing base 130. At this time, the sealing plug 140 is pressed in the space between the inner sleeve 124 of the nasal spray nozzle 120 and the upper end of the fixing base 130, and the lower end of the sealing plug 140, for example, its conical outer surface 141, is pressed against the conical inner surface 134, thereby forming a seal and isolating the liquid medicine pre-filled in the syringe 110 from the external environment (see Fig. 3a).

When it is needed to administer the liquid medicine to the user, the operator (e.g., a licensed physician) rotates the nasal spray nozzle 120 relative to the fixing base 130, so that the sliding block 122 of the nasal spray nozzle 120 slides from the lowered surface 132 onto the raised surface 133 on the guide rail portion 131 of the fixing base 130, thereby causing the nasal spray nozzle 120 to move axially in a direction away from the syringe 110. In this way, the axial pressing force applied to the sealing plug 140 is released, thereby achieving the open state as shown in Fig. 2. At this time, the operator pushes the plunger 112 of the syringe 110, under the pressure of the liquid medicine, the sealing plug 140 is pushed away from the conical inner surface 134, thereby opening the sealing switch (see Fig. 3b), the liquid medicine in the syringe 110 can enter the nasal spray nozzle 120, and then be sprayed out through the nozzle on the nasal spray nozzle 120, and be administered to the user, for example, into the user's nasal cavity.

### <Second Embodiment>

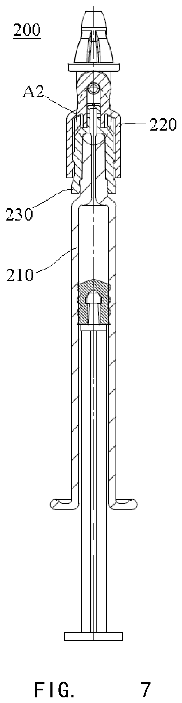

Figs. 7 - 9b show a pre-filled nasal spray device 200 according to a second embodiment of the present application. In absence of contrary description or conflict, the specific structures described above with respect to the first embodiment also apply to the second embodiment. In the following description, the same structures will not be described in detail again, and the structures of the second embodiment that differ from the first embodiment will be specifically described.

Similar to the first embodiment, the pre-filled nasal spray device 200 includes a syringe 210, a nasal spray nozzle 220, and a fixing base 230. The fixing base 230 is connected to the hub of the syringe 210 by a structure such as a snap fit. The nasal spray nozzle 220 and the fixing base 230 may also be connected together by a structure such as a snap fit, and the nasal spray nozzle 220 can rotate relative to the fixing base 230.

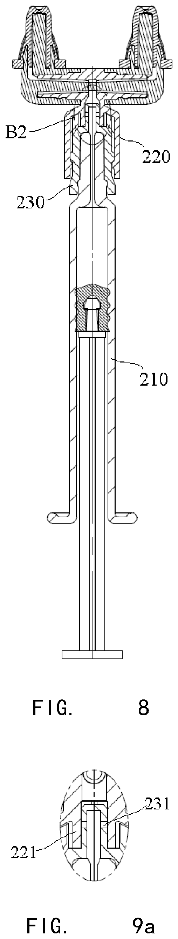

The pre-filled nasal spray device 200 shown in Fig. 7 is in a closed state. With reference to Fig. 9a, it can be seen that at least one side hole 231 is formed in the front end of the fixing base 230. In the exemplary structure shown in the figure, two side holes 231 are included, which are arranged 180° apart from each other. The front end of the fixing base 230 is in an interference fit within the inner sleeve 221 of the nasal spray nozzle 220. In this way, the inner surface of the inner sleeve 221 of the nasal spray nozzle is in sealing contact with the outer surface of the front end of the fixing base 230, particularly the portion including the side holes 231 thereof, so that the side holes 231 are in a closed state.

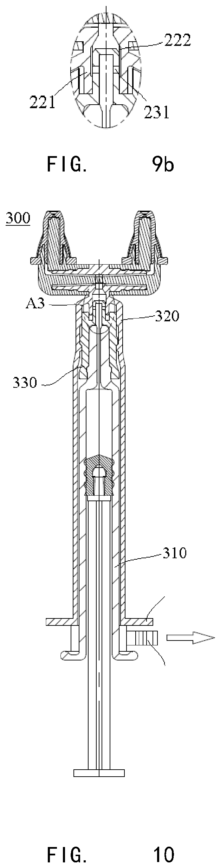

Fig. 8 shows the pre-filled nasal spray device 200 in an open state. Compared with the closed state shown in Fig. 7, the nasal spray nozzle 220 is rotated by a predetermined angle relative to the fixing base 230, for example, by 180°. With reference to Fig. 9b, it can be seen that at least one flow guide groove 222 is provided on an inner surface of the inner sleeve 221, for example, the structure shown in the figure includes two flow guide grooves 222 arranged 180° apart from each other. At this time, after rotating by the predetermined angle, the flow guide grooves 222 in the nasal spray nozzle 220 are aligned with the side holes 231 in the fixing base 230, thereby opening the side holes 231 and releasing the seal of the pre-filled nasal spray device 200.

It can be seen that in the second embodiment, the inner surface of the inner sleeve 221 of the nasal spray nozzle cooperates with the side holes 231 of the fixing base 230 to form a sealing switch for the pre-filled nasal spray device 200.

### <Third Embodiment>

Figs. 10 - 12b show a pre-filled nasal spray device 300 according to a third embodiment of the present application. In absence of contrary description or conflict, the specific structures described above with respect to the first and second embodiments also apply to the third embodiment. In the following description, the same structures will not be described in detail again, and the structures of the third embodiment that differ from the first and second embodiments will be specifically described.

Similar to the previous embodiments, the pre-filled nasal spray device 300 includes a syringe 310, a nasal spray nozzle 320, and a fixing base 330. The fixing base 330 is connected to the hub of the syringe 310 by a structure such as a snap fit. The nasal spray nozzle 320 is connected to the fixing base 330, and is capable of linear movement relative to the fixing base 330 along the axial direction of the pre-filled nasal spray device 300.

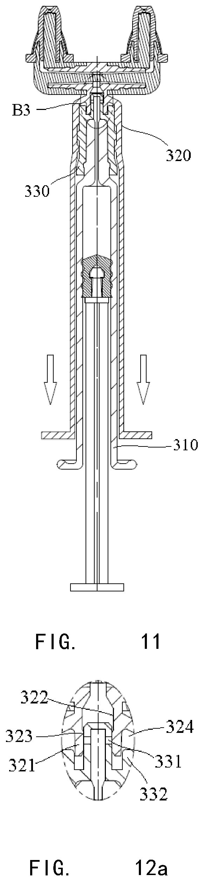

The pre-filled nasal spray device 300 shown in Fig. 10 is in a closed state. With further reference to Fig. 12a, it can be seen that at least one side hole 331 is formed in the front end of the fixing base 330. An inner surface of the inner sleeve 321 of the nasal spray nozzle 320 is formed in a stepped shape, thereby forming a large inner diameter portion 322 with a larger inner diameter and a small inner diameter portion 323 with a smaller inner diameter within the inner sleeve 321. Wherein, the large inner diameter portion 322 is located in front of the small inner diameter portion 323.

The inner diameter of the small inner diameter portion 323 is set to be slightly smaller than the outer diameter of the front end of the fixing base 330, so that the front end of the fixing base 330 can be in an interference fit with the small inner diameter portion 323. Thus, when the front end of the fixing base 330 is fitted in the small inner diameter portion 323, the side holes 331 can be sealed and closed (Fig. 12a).

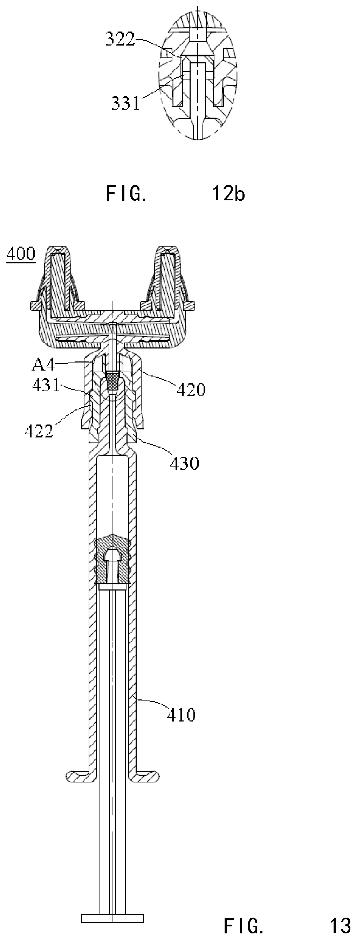

The inner diameter of the large inner diameter portion 322 is set to be larger than the outer diameter of the front end of the fixing base 330. Thus, when the front end of the fixing base 330 is fitted in the large inner diameter portion 322, a gap exists between the front end of the fixing base 330 and the large inner diameter portion 322, thereby opening the side holes 331 (Fig. 12b). Thus, the liquid medicine pre-filled in the syringe 310 can enter the nozzle from the side holes 331 and then be sprayed out.

Preferably, a groove 324 is formed in the inner sleeve 321 of the nasal spray nozzle, and an outer peripheral flange 332 is formed on the fixing base 330. When the nasal spray nozzle 320 is assembled together with the fixing base 330, the outer peripheral flange 332 can fit into the groove 324. Furthermore, the width of the outer peripheral flange 332 is smaller than the width of the groove 324, thereby allowing the nasal spray nozzle 320 to reciprocate axially relative to the fixing base 330.

Preferably, a finger rest plate 325 is also formed on the nasal spray nozzle 320, the finger rest plate 325 may be integrally formed on the nasal spray nozzle 320, or may be formed separately and connected to the nasal spray nozzle 320.

Further preferably, the pre-filled nasal spray device 300 also includes a stop clip 350. In the closed state of the pre-filled nasal spray device 300 shown in Fig. 10, the stop clip 350 is clamped between the finger rest plate 325 of the nasal spray nozzle 320 and the flange on the rear end of the syringe 310, thus preventing the syringe 310 from moving toward the front end relative to the nasal spray nozzle 320.

When the operator is to administer the liquid medicine to the user, the operator removes the stop clip 350. Then, the plunger of the syringe 310 can be pushed towards the front end, under the pressure of the liquid medicine in the syringe 310, the syringe 310 and the fixing base 330 connected to its hub are pushed to move forward, so that the portion of the front end of the fixing base 330 that includes the syringe 310 moves into the large inner diameter portion 322 of the syringe 310, thereby opening the side holes 331.

In addition to the stop clip 350, other structures may also be provided to prevent the relative forward movement of the syringe 310 during storage and transportation. A possible alternative structure is a frangible member provided between the finger rest plate 325 and the rear end flange of the syringe 310, such as a spacing ring with a weakened portion connected between the finger rest plate 325 and the rear end flange of the syringe 310. In use, the operator pushes the plunger of the syringe 310 toward the front end, thereby pushing the syringe 310 forward, under the action of this thrust force, the spacing ring is broken or fractured along the weakened portion, thereby allowing the syringe 310 to move forward.

Furthermore, the frangible member may also be a plurality of frangible struts arranged circumferentially, and these frangible struts are connected between the finger rest plate 325 and the rear end flange of the syringe 310.

The frangible member may be integrally formed on one of the finger rest plate 325 and the rear end flange of the syringe 310. Alternatively, the frangible member may also be formed separately and then connected between the finger rest plate 325 and the rear end flange of the syringe 310.

It can be seen that the side holes 331 on the fixing base 330 cooperate with the large inner diameter portion 322 and the small inner diameter portion 323 in the inner surface of the inner sleeve 321 of the nasal spray nozzle 320 to form a sealing switch.

### <Fourth Embodiment>

Figs. 13 - 15b show a pre-filled nasal spray device 400 according to a fourth embodiment of the present application. In absence of contrary description or conflict, the specific structures described above with respect to the first to third embodiments also apply to the fourth embodiment. In the following description, the same structures will not be described in detail again, and the structures of the fourth embodiment that differ from the first to third embodiments will be specifically described.

In the fourth embodiment, the pre-filled nasal spray device 400 includes a syringe 410, a nasal spray nozzle 420, and a fixing base 430. The fixing base 430 is connected to the hub of the syringe 410 by a structure such as a snap fit. The nasal spray nozzle 420 is connected to the fixing base 430 and can move relative to the fixing base 430 along the axial direction of the pre-filled nasal spray device 400. Specifically, as shown in Fig. 13, a protruding ring 431 is formed on the outer surface of the fixing base 430, and a groove 422 is formed on the inner surface of the outer sleeve of the nasal spray nozzle 420, the protruding ring 431 fits into the groove 422, and the fit between the protruding ring 431 and the groove 422 allows relative movement between the nasal spray nozzle 420 and the fixing base 430 along the axial direction of the pre-filled nasal spray device 400.

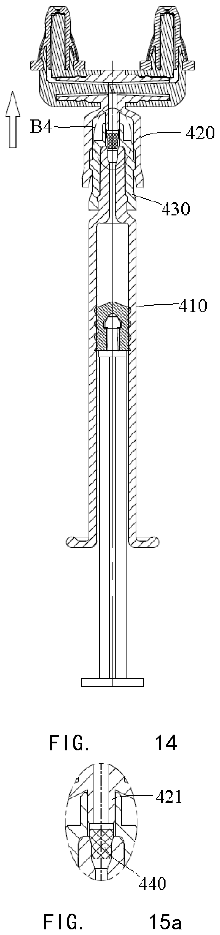

The pre-filled nasal spray device 400 further includes a sealing plug 440, the sealing plug 440 is located in the space in the front end of the fixing base 430, and can be inserted into the liquid outlet on the hub of the syringe 410. As shown in Fig. 15a, when the pre-filled nasal spray device 400 is in the closed state shown in Fig. 13, the nasal spray nozzle 420 is in a rearward position relative to the fixing base 430, wherein the inner sleeve 421 of the nasal spray nozzle 420 presses against the sealing plug 440, keeping the sealing plug 440 in the state of being inserted in the liquid outlet of the hub of the syringe 410.

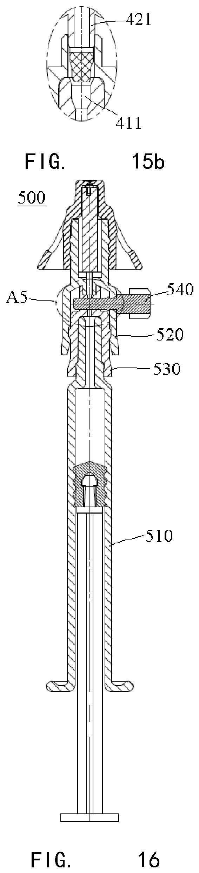

In use, the operator pulls the nasal spray nozzle 420 forward relative to the fixing base 430 (as shown by the arrow in Fig. 14), thereby releasing the pressing of the sealing plug 440 by the inner sleeve 421 of the nasal spray nozzle 420 (see Fig. 15b). Then, the plunger of the syringe 410 is pushed, and under the pressure of the liquid medicine in the syringe 410, the sealing plug 440 is pushed open, so that the medicine can be administered outward.

It can be seen that in the fourth embodiment, the sealing plug 440 cooperates with the inner sleeve 421 of the nasal spray nozzle 420 to function as a sealing switch.

### <Fifth Embodiment>

Figs. 16 - 18b show a pre-filled nasal spray device 500 according to a fifth embodiment of the present application. In absence of contrary description or conflict, the specific structures described above with respect to the first to fourth embodiments also apply to the fifth embodiment. In the following description, the same structures will not be described in detail again, and the structures of the fifth embodiment that differ from the first to fourth embodiments will be specifically described.

In the fifth embodiment, the pre-filled nasal spray device 500 includes a syringe 510, a nasal spray nozzle 520, and a fixing base 530. The fixing base 530 is connected to the hub of the syringe 510, and the nasal spray nozzle 520 is connected to the fixing base 530. The connection between the above-mentioned components can be achieved, for example, by a snap ring-groove structure or the like.

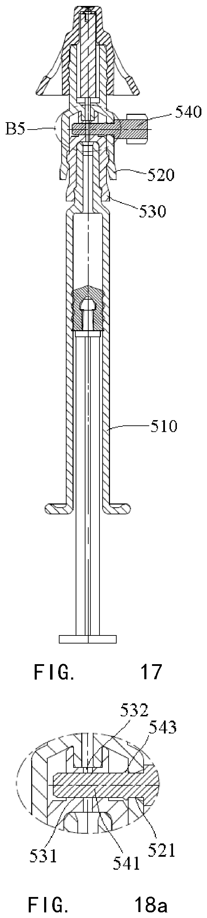

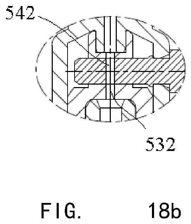

The pre-filled nasal spray device 500 further includes a knob 540 disposed in the front end of the fixing base 530. Specifically referring to Fig. 18a and Fig. 18b, the knob 540 includes a rod portion 541, a transverse hole 531 is formed in the front end of the fixing base 530, the transverse hole 531 extends in a direction substantially perpendicular to the axial direction of the pre-filled nasal spray device 500, the rod portion 541 is inserted into the transverse hole 531. In addition, a side opening 521 is also formed in the side portion of the nasal spray nozzle 520, the rod portion 541 of the knob 540 can be inserted into the side opening 521, and then inserted into the transverse hole 531 of the fixing base 530.

As shown in Fig. 18b, a through hole 542 is formed in the rod portion 541 of the knob 540. The knob 540 is rotatable relative to the fixing base 530, thereby switching between a position where the through hole 542 is aligned with and a position where it is offset from a longitudinal channel 532 in the fixing base 530.

Preferably, as shown in Fig. 18a and Fig. 18b, a protruding ring 543 is also formed on the rod portion 541, which helps to keep the knob 540 in the state of being inserted in the transverse hole 531 of the fixing base 530 and prevent it from coming out.

In the closed state shown in Fig. 16 and Fig. 18a, the through hole 542 of the knob 540 is offset from the longitudinal channel 532 in the front end of the fixing base 530, thereby isolating the liquid medicine in the syringe 510 from the external environment. In use, the operator rotates the knob 540 so that the through hole 542 in the knob 540 is aligned with the longitudinal channel 532 in the fixing base 530, as shown in Fig. 18b, thereby communicating the interior of the syringe 510 with the external environment, so that the liquid medicine can be sprayed outward.

It can be seen that in the fifth embodiment, the knob 540 functions as a sealing switch.

## Claims

1. A pre-filled nasal spray device having a syringe, a nasal spray nozzle and a fixing base, the fixing base being mounted on the syringe, the nasal spray nozzle being connected to the fixing base, wherein a liquid channel from an internal chamber of the syringe to the nasal spray nozzle through the fixing base is formed in the pre-filled nasal spray device, **characterized in that**,
a sealing switch is provided in the liquid channel, the sealing switch being switchable between an open state and a closed state, in the open state, the internal chamber of the syringe communicates with the outside through the liquid channel, in the closed state, the internal chamber of the syringe is sealed and isolated from the outside.

2. The pre-filled nasal spray device according to claim 1, **characterized in that** the sealing switch comprises a sealing plug, a sealing surface is formed on a front end of the fixing base or on a hub of the syringe, and the sealing plug is capable of forming a sealing fit with the sealing surface.

3. The pre-filled nasal spray device according to claim 2, **characterized in that** the nasal spray nozzle is movable relative to the fixing base along an axial direction of the pre-filled nasal spray device between a closed position and an open position, in the closed position, the nasal spray nozzle presses the sealing plug against the sealing surface, in the open position, the nasal spray nozzle releases pressing against the sealing plug.

4. The pre-filled nasal spray device according to claim 3, **characterized in that** the fixing base comprises a guide rail portion, a top surface of the guide rail portion comprises a lowered surface and a raised surface;
at least one sliding block is formed on an outer surface of an outer sleeve of the nasal spray nozzle, the sliding block resting on the top surface of the guide rail portion;
wherein the nasal spray nozzle is rotatable relative to the fixing base, so that the sliding block contacts the lowered surface and the raised surface alternatively, when the sliding block contacts the lowered surface, the nasal spray nozzle is in the closed position relative to the fixing base, when the sliding block contacts the raised surface, the nasal spray nozzle is in the open position relative to the fixing base.

5. The pre-filled nasal spray device according to claim 3, **characterized in that** the nasal spray nozzle is configured to be linearly movable relative to the fixing base along the axial direction of the pre-filled nasal spray device between the closed position and the open position.

6. The pre-filled nasal spray device according to claim 2, **characterized in that** the sealing surface is a conical inner surface formed in the front end of the fixing base or in the hub of the syringe.

7. The pre-filled nasal spray device according to claim 1, **characterized in that** the sealing switch comprises an opening, the sealing switch is movable between a closed position and an open position, in the closed position, the opening is closed, in the open position, the opening is opened.

8. The pre-filled nasal spray device according to claim 7, **characterized in that** the opening is at least one side hole formed in a front end of the fixing base.

9. The pre-filled nasal spray device according to claim 8, **characterized in that** the nasal spray nozzle comprises an inner sleeve, an inner diameter of the inner sleeve of the nasal spray nozzle is set to be smaller than an outer diameter of a portion of the front end of the fixing base where the side hole is formed, so that the portion formed with the side hole of the front end is in interference fit with the inner sleeve of the nasal spray nozzle, and at least one flow guide groove is formed in the inner sleeve of the nasal spray nozzle;
wherein the nasal spray nozzle is rotatable relative to the fixing base between the closed position and the open position, in the closed position, the side hole is offset from the flow guide groove, in the open position, the side hole is aligned with the flow guide groove.

10. The pre-filled nasal spray device according to claim 8, **characterized in that** the nasal spray nozzle comprises an inner sleeve, a large inner diameter portion and a small inner diameter portion are formed in the inner sleeve of the nasal spray nozzle, an inner diameter of the small inner diameter portion is smaller than an outer diameter of a portion formed with the side hole of the front end of the fixing base, so that the portion formed with the side hole of the front end is in interference fit with the small inner diameter portion, and an inner diameter of the large inner diameter portion is larger than the outer diameter of the portion formed with the side hole of the front end of the fixing base;
wherein the nasal spray nozzle is linearly movable relative to the fixing base along the axial direction of the pre-filled nasal spray device between the closed position and the open position, in the closed position, the portion formed with the side hole of the front end of the fixing base is in interference fit with the small inner diameter portion, in the open position, the portion formed with the side hole of the front end of the fixing base is located within the large inner diameter portion.

11. The pre-filled nasal spray device according to claim 10, **characterized in that** a groove is formed in the nasal spray nozzle, and an outer peripheral flange is formed on the fixing base, when the nasal spray nozzle is connected to the fixing base, the outer peripheral flange fits into the groove, wherein a width of the groove is greater than a width of the outer peripheral flange, so as to allow a linear movement of the nasal spray nozzle relative to the fixing base.

12. The pre-filled nasal spray device according to claim 7, **characterized in that** the sealing switch comprises a knob comprises a rod portion, and the opening is a through hole formed in the rod portion;
a transverse hole is formed in the fixing base, the transverse hole extending in a direction perpendicular to the axial direction of the pre-filled nasal spray device, the rod portion being inserted into the transverse hole of the fixing base;
wherein the knob is rotatable between a closed position and an open position, in the closed position, the through hole is offset from a longitudinal channel of the fixing base, in the open position, the through hole is aligned with the longitudinal channel.

13. The pre-filled nasal spray device according to claim 1, **characterized in that** a finger rest plate is integrally formed on or connected to the nasal spray nozzle or the fixing base.

14. The pre-filled nasal spray device according to claim 13, **characterized in that** a stop clip is provided between the finger rest plate and a flange on a rear end of the syringe.

15. The pre-filled nasal spray device according to claim 13, **characterized in that** at least one frangible member is provided between the finger rest plate and a flange on a rear end of the syringe.
